# EUROPEAN PATENT APPLICATION

(11) **EP 4 748 357 A1**
(43) Date of publication of application: **27.05.2026**
(21) Application number: 24841965.7
(22) Date of filing: 27.03.2024
(51) Int. Cl.: A61F 13/496, A61F 13/15

(54) **APPARATUS AND METHOD FOR MANUFACTURING PAPER DIAPER CORE, AND PAPER DIAPER CORE**

(30) Priority: 17.07.2023 CN 202310875315
(71) Applicant: SHANDONG THRIVE MATERNITY AND INFANT PRODUCTS CO., LTD, Shandong 277500 (CN)
(72) Inventor: SUN, Feng, Zaozhuang, Shandong 277500 (CN); LIANG, Jinpan, Zaozhuang, Shandong 277500 (CN); WANG, Xishan, Zaozhuang, Shandong 277500 (CN)
(74) Representative: Cabinet Beau de Loménie
(86) International application number: PCT/CN2024/084048
(87) International publication number: WO 2025/015964

(57) **Abstract**

The present disclosure relates to an apparatus and a method for manufacturing a diaper core, and a diaper core. The apparatus for manufacturing a diaper core comprises: a forming unit capable of rotating around its own axis and having a hollowed-out area and a pattern block on its circumferential surface; a first conveying unit capable of supplying a first fluffy fabric to the forming unit and enabling the first fluffy fabric to be wound on the circumferential surface of the forming unit, wherein the first fluffy fabric can rotate along with the forming unit, and has a first area corresponding to the hollowed-out area and a second area corresponding to the pattern block; a feeding unit capable of supplying polymer particles to the first fluffy fabric wound on the forming unit; and a negative pressure unit capable of adsorbing the polymer particles onto the first area of the first fluffy fabric. According to the present disclosure, a second area having no polymer particles can be formed on the fluffy fabric of the diaper core, thereby improving the urine absorption effect of the fluffy fabric of the diaper core.

## Description

### Technical Field

The present disclosure relates to the field of diapers, particularly to an apparatus and a method for manufacturing a diaper core, and a diaper core.

### Background Art

Diapers are widely used by infants, young children, and patients who are unable to care for themselves. Existing diapers have a smooth surface, which easily causes urine and other substances to accumulate on the surface (i.e., the surface layer) of the diaper. The fluffy fabric inside the diaper, which can absorb large amounts of urine, cannot be fully utilized, resulting in poor and slow absorption of urine. This causes urine to accumulate on the surface layer, which is in direct contact with the user's skin, leading to a poor user experience.

The information disclosed in the background section of the present disclosure is only intended to enhance an understanding of the general background of the present disclosure, and should not be construed as an admission or suggestion in any form that such information constitutes prior art known to those skilled in the art.

### Summary

The present disclosure aims to provide an apparatus and a method for manufacturing a diaper core, and a diaper core. According to the apparatus and the method for manufacturing a diaper core of the present disclosure, a second area having no polymer particles can be formed on the fluffy fabric of the diaper core, thereby improving the urine absorption effect of the fluffy fabric of the diaper core.

According to a first aspect of the present disclosure, there is provided an apparatus for manufacturing a diaper core, comprising: a forming unit capable of rotating around its own axis and having a hollowed-out area and a pattern block on its circumferential surface; a first conveying unit capable of supplying a first fluffy fabric to the forming unit and enabling the first fluffy fabric to be wound on the circumferential surface of the forming unit, wherein the first fluffy fabric can rotate along with the forming unit, and has a first area corresponding to the hollowed-out area and a second area corresponding to the pattern block; a feeding unit capable of supplying polymer particles to the first fluffy fabric wound on the forming unit; and a negative pressure unit capable of adsorbing the polymer particles onto the first area of the first fluffy fabric.

Preferably, the apparatus for manufacturing a diaper core further comprises a second conveying unit capable of supplying a wrapping fabric to the forming unit.

Preferably, the apparatus for manufacturing a diaper core further comprises a third conveying unit capable of supplying a second fluffy fabric to the forming unit.

Preferably, the apparatus for manufacturing a diaper core further comprises a fourth conveying unit capable of supplying non-woven fabric.

Preferably, the feeding unit comprises: a first feeding hopper capable of supplying first polymer particles to the first fluffy fabric wound on the forming unit; and a second feeding hopper capable of supplying second polymer particles to the first fluffy fabric wound on the forming unit.

Preferably, the pattern block has a curvature.

Preferably, the pattern block is integrated with the hollowed-out area.

Preferably, the circumferential surface of the forming unit has a through hole, and the pattern block has a plug-in rod corresponding to the through hole and inserted into the through hole so that the pattern block is detachably installed on the circumferential surface of the forming unit.

Preferably, the feeding unit is arranged above or diagonally above the circumferential surface of the forming unit.

Preferably, the forming unit comprises: a shaft; and a housing rotatably mounted to the shaft.

According to a second aspect of the present disclosure, there is provided a method for manufacturing a diaper core, comprising: supplying, through a first conveying unit, a first fluffy fabric to a forming unit that is rotatable around its own axis and having a hollowed-out area and a pattern block on its circumferential surface, wherein the first fluffy fabric can rotate along with the forming unit, and has a first area corresponding to the hollowed-out area and a second area corresponding to the pattern block; supplying, through a feeding unit, polymer particles to the first fluffy fabric wound on the forming unit; and adsorbing, through a negative pressure unit, the polymer particles onto the first area of the first fluffy fabric.

Preferably, before supplying, through the first conveying unit, the first fluffy fabric to the forming unit, the method for manufacturing a diaper core further comprises: supplying, through a second conveying unit, a wrapping fabric to the forming unit; wherein the first fluffy fabric is disposed above the wrapping fabric.

Preferably, after supplying, through the first conveying unit, the first fluffy fabric to the forming unit, the method for manufacturing a diaper core further comprises: supplying, through a third conveying unit, a second fluffy fabric to the forming unit; wherein the second fluffy fabric is disposed above the first fluffy fabric.

Preferably, after adsorbing, through the negative pressure unit, the polymer particles onto the first area of the first fluffy fabric, the method for manufacturing a diaper core further comprises: supplying non-woven fabric through a fourth conveying unit, so that the non-woven fabric is disposed above the first fluffy fabric.

Preferably, the feeding unit comprises: a first feeding hopper capable of supplying first polymer particles to the first fluffy fabric wound on the forming unit; and a second feeding hopper capable of supplying second polymer particles to the first fluffy fabric wound on the forming unit.

Preferably, the feeding unit is arranged above or diagonally above the circumferential surface of the forming unit.

According to a third aspect of the present disclosure, there is provided a diaper core manufactured by the apparatus for manufacturing a diaper core according to the first aspect.

According to a fourth aspect of the present disclosure, there is provided a diaper core manufactured by the method for manufacturing a diaper core according to the second aspect.

According to the apparatus and the method for manufacturing a diaper core of the present disclosure, a second area having no polymer particles can be formed on the fluffy fabric of the diaper core, thereby improving the urine absorption effect of the fluffy fabric of the diaper core.

The units of the present disclosure have other characteristics and advantages that will be apparent from the accompanying drawings and subsequent specific embodiments incorporated herein, or will be described in detail in the accompanying drawings and subsequent specific embodiments, which together explain the specific principles of the present disclosure.

### Brief Description of Drawings

Figure 1 is a structural schematic diagram of an apparatus for manufacturing a diaper core according to an embodiment of the present disclosure;
Figure 2A is a schematic diagram of the first state of a forming unit;
Figure 2B is a schematic diagram of the second state of a forming unit;
Figure 2C is a schematic diagram of the third state of a forming unit;
Figure 3A is a schematic diagram I of the positional relationship between a forming unit and a feeding unit;
Figure 3B is a schematic diagram II of the positional relationship between a forming unit and a feeding unit;
Figure 4A is a cross-sectional view of a diaper core in Example 1 of the present disclosure;
Figure 4B is a cross-sectional view of a diaper core in Example 2 of the present disclosure;
Figure 5A is a cross-sectional view of a diaper core in Example 3 of the present disclosure;
Figure 5B is a cross-sectional view of a diaper core in Example 4 of the present disclosure;
Figure 6A is a schematic diagram of the first type of a second area on a first fluffy fabric;
Figure 6B is a schematic diagram of the second type of a second area on a first fluffy fabric;
Figure 6C is a schematic diagram of the third type of a second area on a first fluffy fabric;
Figure 6D is a schematic diagram of the fourth type of a second area on a first fluffy fabric;
Figure 6E is an unfolded view of a wrapping fabric;
Figure 7 is a schematic flowchart illustrating a first method for manufacturing a diaper core provided by the present disclosure;
Figure 8A is a schematic flowchart illustrating a second method for manufacturing a diaper core provided by the present disclosure;
Figure 8B is a schematic flowchart illustrating a third method for manufacturing a diaper core provided by the present disclosure;
Figure 8C is a schematic flowchart illustrating a fourth method for manufacturing a diaper core provided by the present disclosure;
Figure 9A is a schematic flowchart illustrating a fifth method for manufacturing a diaper core provided by the present disclosure;
Figure 9B is a schematic flowchart illustrating a sixth method for manufacturing a diaper core provided by the present disclosure;
Figure 9C is a schematic flowchart illustrating a seventh method for manufacturing a diaper core provided by the present disclosure.

### List of reference numerals:

| | | | |
|---|---|---|---|
| 100: | forming unit | 101: | hollowed-out area |
| 102: | pattern block | 103: | shaft |
| 104: | housing | 201: | first conveying unit |
| 300: | feeding unit | 301: | first feeding hopper |
| 302: | second feeding hopper | 400: | negative pressure unit |
| 202: | second conveying unit | 203: | third conveying unit |
| 204: | fourth conveying unit | 801: | first fluffy fabric |
| 802: | first area | 803: | second area |
| 804: | second fluffy fabric | 901: | wrapping fabric |
| 902: | non-woven fabric | 903: | long side. |

It should be understood that the accompanying drawings are not necessarily drawn to scale, but rather present simplified representations of various features to illustrate the basic principles of the present disclosure. The specific design features disclosed in the present disclosure, including specific dimensions, directions, positions, and shapes, will be partially determined by the specific application and usage environment.

In these drawings, like reference numerals refer to the same or equivalent parts of the present disclosure throughout the drawings.

### Detailed Description of Embodiments

Reference will now be made in detail to various embodiments of the present disclosure, examples of which are presented in the accompanying drawings and described as follows. Although the present disclosure is described in conjunction with exemplary embodiments, it should be understood that this description is not intended to limit the present disclosure to these exemplary embodiments. On the contrary, the present disclosure aims to cover not only these exemplary embodiments, but also various substitutions, modifications, equivalents, and other embodiments that can be included within the spirit and scope of the present disclosure as defined by the appended claims.

It should be understood that when a component is referred to as being "connected to" another component, the component may be directly connected to the other component or there may be intermediate components. On the contrary, when a component is referred to as being "directly connected" to another component, there is usually no intermediate component.

When a component is referred to as being "above" or "on" another component, the component may be in contact with the other component or there may be intermediate components.

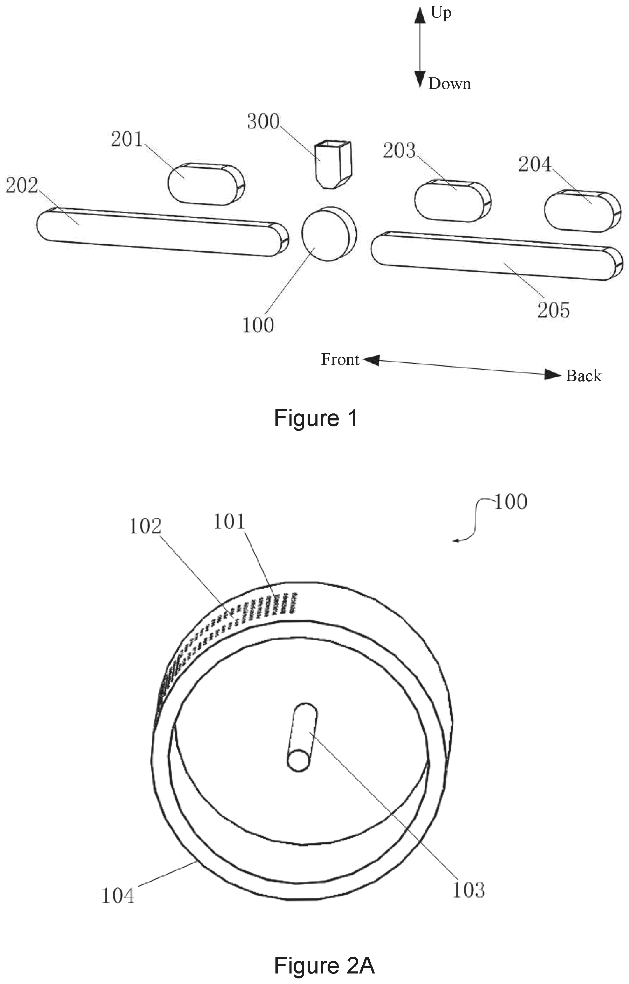

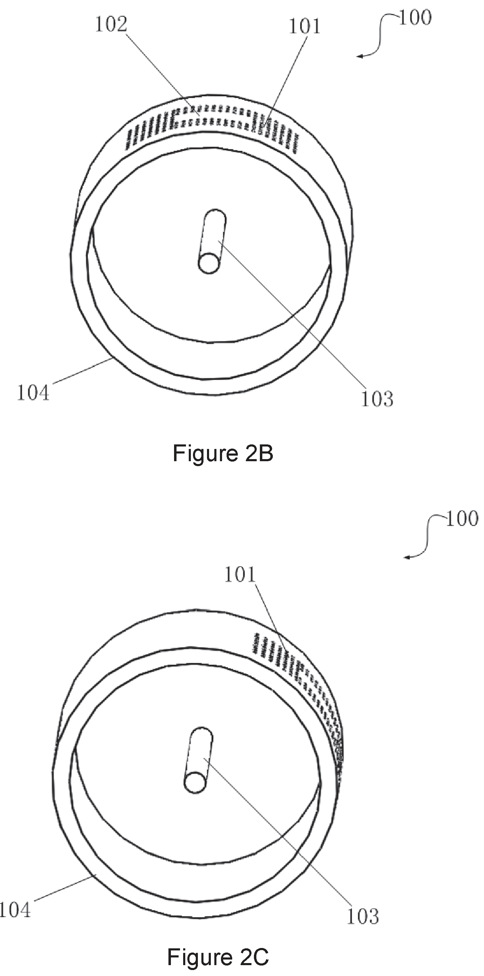

Figure 1 is a structural schematic diagram of an apparatus for manufacturing a diaper core according to an embodiment of the present disclosure; Figure 2A is a schematic diagram of the first state of a forming unit; Figure 2B is a schematic diagram of the second state of a forming unit; Figure 2C is a schematic diagram of the third state of a forming unit.

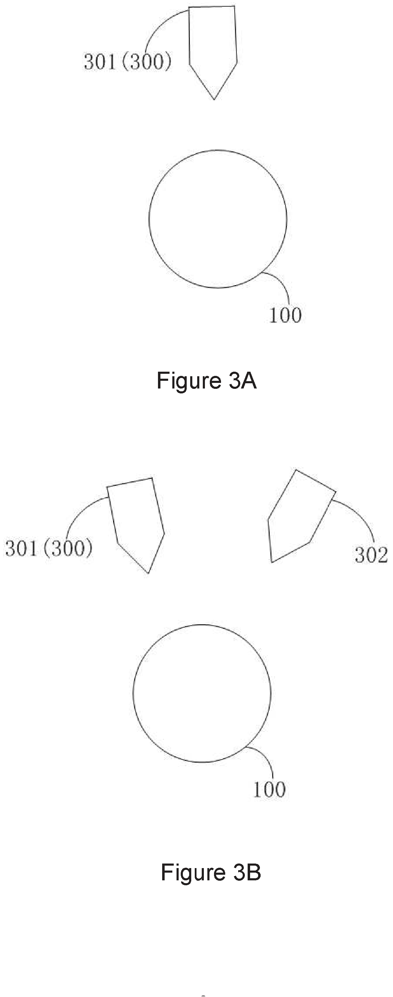

Figure 3A is a schematic diagram I of the positional relationship between a forming unit and a feeding unit, wherein the feeding unit 300 only includes one first feeding hopper 301; Figure 3B is a schematic diagram II of the positional relationship between a forming unit and a feeding unit, wherein the feeding unit 300 includes a first feeding hopper 301 and a second feeding hopper 302.

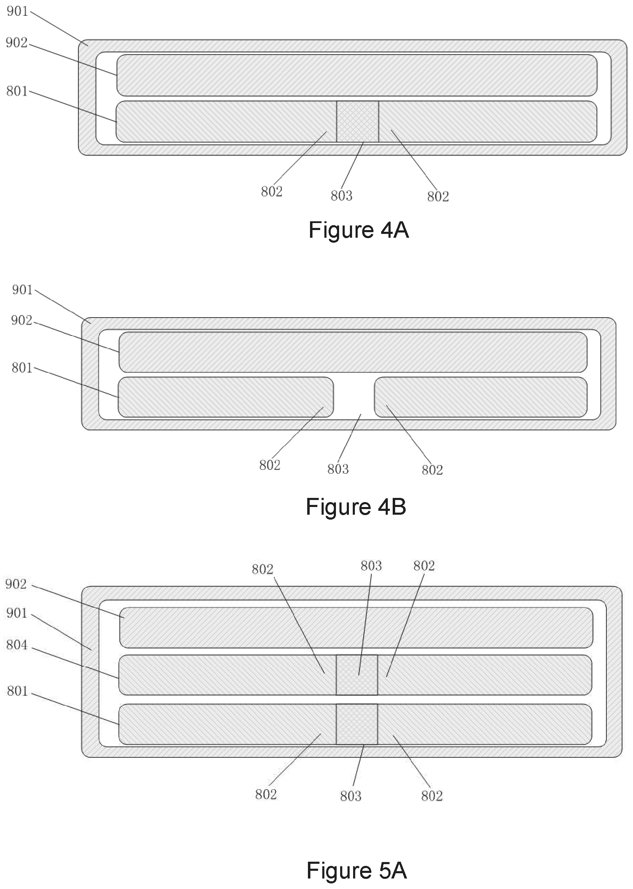

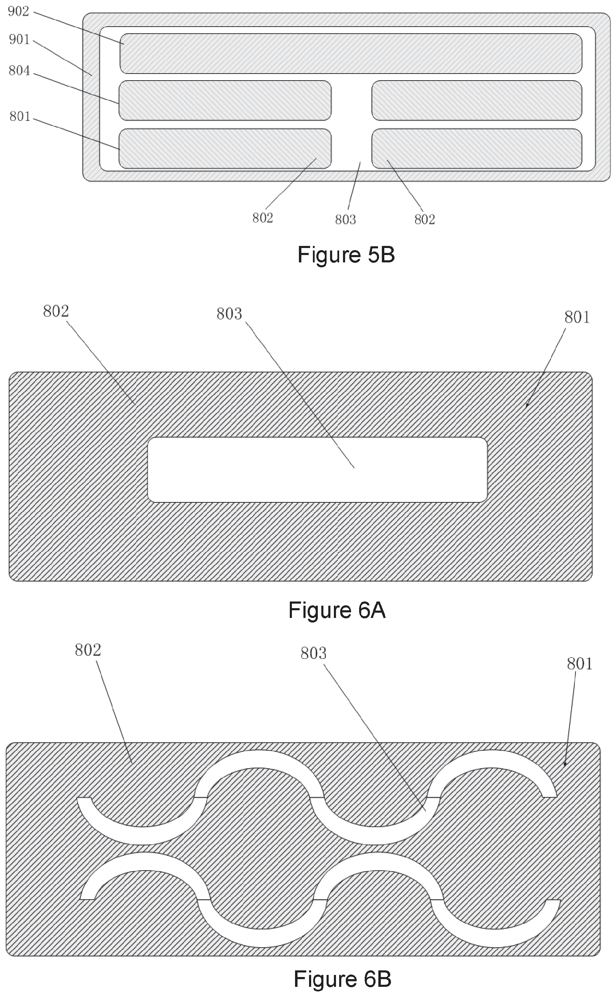

Figure 4A is a cross-sectional view of a diaper core in Example 1 of the present disclosure, and Figure 4B is a cross-sectional view of a diaper core in Example 2 of the present disclosure. The diaper core in both Figure 4A and Figure 4B includes only one layer of fluffy fabric, namely, a first fluffy fabric 801. Figure 5A is a cross-sectional view of a diaper core in Example 3 of the present disclosure, and Figure 5B is a cross-sectional view of a diaper core in Example 4 of the present disclosure. The diaper core in both Figure 5A and Figure 5B includes two layers of fluffy fabric, namely, a first fluffy fabric 801 and a second fluffy fabric 804. The second area 803 in Figures 4A and 5A is a solid, while the second area 803 in Figures 4B and 5B is an opening.

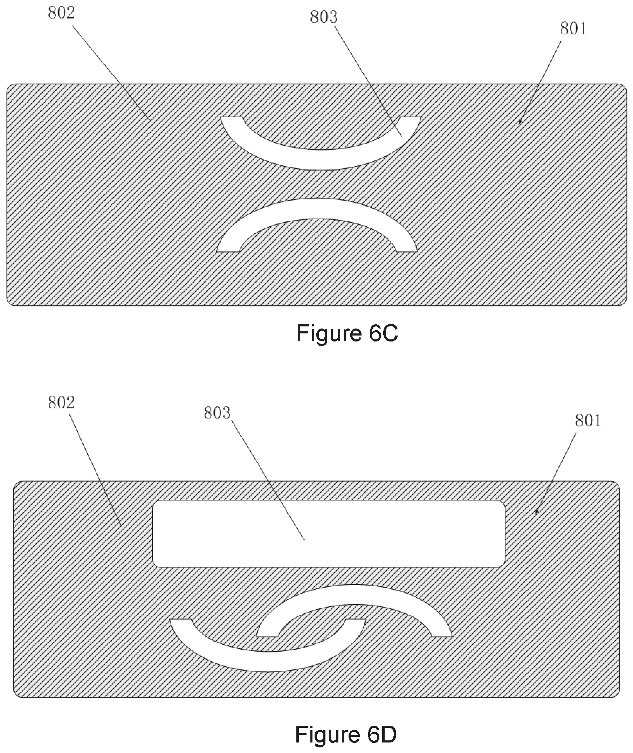

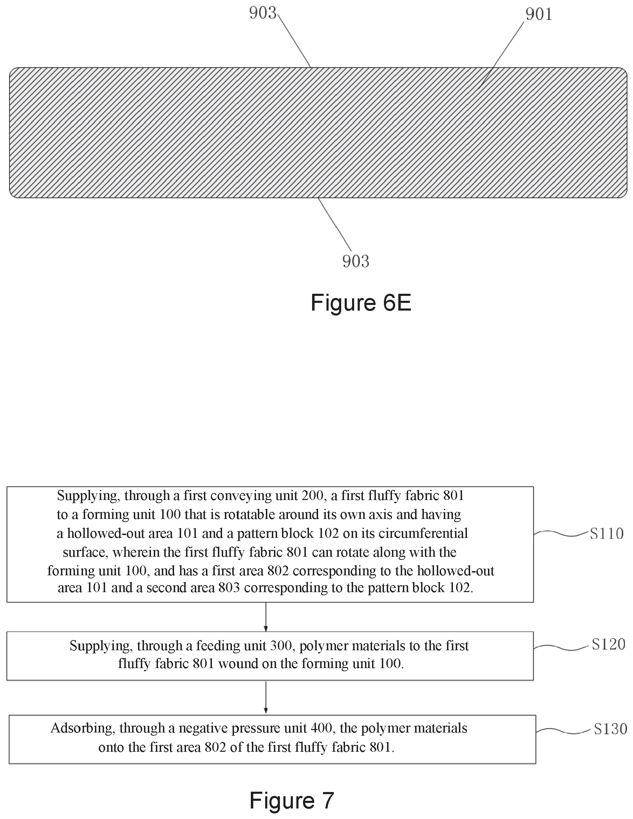

Figure 6A is a schematic diagram of the first type of a second area on a first fluffy fabric; Figure 6B is a schematic diagram of the second type of a second area on a first fluffy fabric; Figure 6C is a schematic diagram of the third type of a second area on a first fluffy fabric; and Figure 6D is a schematic diagram of the fourth type of a second area on a first fluffy fabric. Figures 6A, 6B, 6C, and 6D illustrate four different shapes of the second area 803. Figure 6E is an unfolded diagram of the wrapping fabric.

As shown in Figure 1, an apparatus for manufacturing a diaper core in an embodiment of the present disclosure comprises a forming unit 100, a first conveying unit 201, a feeding unit 300, and a negative pressure unit 400.

The forming unit 100 is rotatable around its own axis, and is provided with a hollowed-out area 101 and a pattern block 102 on its circumferential surface (see Figure 2A for reference). The hollowed-out area 101 surrounds the pattern block 102.

The first conveying unit 201 can supply the first fluffy fabric 801 to the forming unit 100, enabling the first fluffy fabric 801 to be wound on the circumferential surface of the forming unit 100. The first fluffy fabric 801 is rotatable along with the forming unit 100, and has a first area 802 corresponding to the hollowed-out area 101 and a second area 803 corresponding to the pattern block 102 (see Figure 6A, Figure 6B, Figure 6C, and Figure 6D for reference).

The feeding unit 300 can supply polymer particles to the first fluffy fabric 801 wound on the forming unit 100. There can be only one type of polymer particle, but it is not limited to only one type; for example, it may include first polymer particles and second polymer particles described later. Specifically, the feeding unit 300 is positioned above (see Figure 3A for reference) or diagonally above (see Figure 3B for reference) the circumferential surface of the forming unit.

The negative pressure unit 400 can adsorb the polymer particles onto the first area 802 of the first fluffy fabric 801.

According to the apparatus for manufacturing a diaper core of the present disclosure, a second area having no polymer particles can be formed on the fluffy fabric of the diaper core, and the second area having no polymer particles facilitates the diffusion of urine from the non-woven fabric above the fluffy fabric to the fluffy fabric, thereby improving the urine absorption effect of the fluffy fabric of the diaper core.

In an exemplary embodiment, as shown in Figure 1, the apparatus for manufacturing a diaper core according to an embodiment of the present disclosure further comprises a second conveying unit 202, which can supply a wrapping fabric 901 to the forming unit 100 (see Figure 4A for reference). The wrapping fabric 901 can wrap around the first fluffy fabric 801 and the non-woven fabric 902 described later.

In an exemplary embodiment, as shown in Figure 1, the apparatus for manufacturing a diaper core according to an embodiment of the present disclosure further comprises a third conveying unit 203, which can supply a second fluffy fabric 804 that is arranged above the first fluffy fabric 801 to the forming unit 100 (see Figure 4A for reference), and the wrapping fabric 901 can wrap around the first fluffy fabric 801, the second fluffy fabric 804, and the non-woven fabric 902 described later.

In an exemplary embodiment, as shown in Figure 1, the apparatus for manufacturing a diaper core according to an embodiment of the present disclosure further comprises a fourth conveying unit 204, which can supply non-woven fabric 902 (Figures 4A, 4B, 5A, and 5B). The non-woven fabric 902 is used to diffuse urine towards the lower part and both ends of the core to facilitate the absorption by polymer particles.

The non-woven fabric 902 can be selected from spunlace fabric, spunbond fabric, or spunmelt fabric.

In an exemplary embodiment, as shown in Figure 1, the apparatus for manufacturing a diaper core according to an embodiment of the present disclosure further comprises a fifth conveying unit 205, and the formed diaper core is conveyed to the next assembly line through the fifth conveying unit 205.

In an embodiment, as shown in Figure 1, the first conveying unit 201 is positioned above the second conveying unit 202. In another embodiment, the first conveying unit 201 and the second conveying unit 202 are located on the same level. The positional relationship between the first conveying unit 201 and the second conveying unit 202 here is not limited to this, and can be any relationship in the prior art as long as it can achieve the above-mentioned functions.

In an embodiment, as shown in Figure 1, the third conveying unit 203 is positioned above the fifth conveying unit 205. In another embodiment, the third conveying unit 203 and the fifth conveying unit 205 are located on the same level. The positional relationship between the third conveying unit 203 and the fifth conveying unit 205 here is not limited to this, and can be any relationship in the prior art as long as it can achieve the above-mentioned functions.

In an embodiment, as shown in Figure 1, the fourth conveying unit 204 is positioned above the fifth conveying unit 205. In another embodiment, the fourth conveying unit 204 and the fifth conveying unit 205 are located on the same level. The positional relationship between the fourth conveying unit 204 and the fifth conveying unit 205 here is not limited to this, and can be any relationship in the prior art as long as it can achieve the above-mentioned functions.

In an embodiment, as shown in Figure 3A, the feeding unit 300 only includes a first feeding hopper 301, which only provides the first polymer particles to the first fluffy fabric 801.

In another embodiment, as shown in Figures 1 and 4A, the feeding unit 300 includes a first feeding hopper 301 and a second feeding hopper 302.

The first feeding hopper 301 is filled with the first polymer particles, and can supply the first polymer particles to the first fluffy fabric 801 wound on the forming unit 100.

The second feeding hopper 302 is filled with second polymer particles, and can supply the second polymer particles to the first fluffy fabric 801 wound on the forming unit 100.

The operator can open only the first feeding hopper 301 without opening the second feeding hopper 302 to provide only the first polymer particles, or open only the second feeding hopper 302 without opening the first feeding hopper 301 to provide only the second polymer particles, or open both the first and second feeding hoppers 301 and 302 simultaneously to provide both the first and second polymer particles.

In an exemplary embodiment, as shown in Figure 2B, the pattern block 102 has a curvature such that the polymer particles falling on the second area 803 corresponding to the pattern block 102 fall from the second area 803 to the first area 802 corresponding to the hollowed-out area 101.

In an embodiment, the pattern block 102 is integrated with the hollowed-out area 101.

In another embodiment, the circumferential surface of the forming unit 100 has a through hole (not illustrated in the figure), and the pattern block 102 has a plug-in rod (not illustrated in the figure) corresponding to the through hole, and the plug-in rod is inserted into the through hole so that the pattern block 102 can be detachably installed to the circumferential surface of the forming unit 100.

The shape of the second area 803 is determined by the shape of the pattern block 102. When it is necessary to change the shape of the second area 803, it only needs to replace the pattern block 102 with a different shape.

The installation method using the through hole and the plug-in rod is only an optional approach. In other embodiments, other installation methods can also be chosen to allow the pattern block 102 to be detachably installed on the circumferential surface of the forming unit 100.

In an exemplary embodiment, as shown in Figures 2A, 2B, and 2C, the forming unit 100 includes a shaft 103 and a housing 104. The housing 104 is rotatably mounted to the shaft 103. Figures 2A, 2B, and 2C do not illustrate the complete structure of the forming unit 100, but only show the approximate positional relationship between the shaft 103 and the housing 104.

In an exemplary embodiment, the interior of the shaft 103 is hollow and is communicated with the interior of housing 104. The negative pressure unit 400 includes a negative pressure generating device and a connecting pipe (neither shown). One end of the connecting pipe is communicated with the negative pressure generating device, and the other end of the connecting pipe is communicated with the interior of the shaft, thereby connecting the negative pressure generating device to the interior of the housing 104, so that negative pressure can be generated inside the housing 104.

As shown in Figure 7, an embodiment of the present disclosure also provides a method for manufacturing a diaper core, comprising:
Step S110, supplying, through a first conveying unit 201, a first fluffy fabric 801 to a forming unit 100 that is rotatable around its own axis and having a hollowed-out area 101 and a pattern block 102 on its circumferential surface, wherein the first fluffy fabric 801 can rotate along with the forming unit 100, and has a first area 802 corresponding to the hollowed-out area 101 and a second area 803 corresponding to the pattern block 102.
Step S120, supplying, through a feeding unit 300, polymer particles to the first fluffy fabric 801 wound on the forming unit 100. The forming unit 100 rotates from the state of Figure 2A to the state of Figure 2B, and then to the state of Figure 2C, wherein when the forming unit 100 rotates to the state of Figure 2B, the pattern block 102 of the forming unit 100 is at the highest point. At this time, the polymer particles supplied by the feeding unit 300 fall onto the first area 802 and the second area 803 of the first fluffy fabric 801. Because the pattern block 102 has curvature and at its highest point, the polymer particles that fall onto the second area 803 corresponding to the pattern block 102 fall from the second area 803 to the first area 802 corresponding to the hollowed-out area 101. At the same time, the negative pressure generated by the negative pressure unit 400 causes the polymer particles to be adsorbed on the first area 802.
Step S130, adsorbing, through the negative pressure unit 400, the polymer particles onto the first area 802 of the first fluffy fabric 801.

According to the method for manufacturing a diaper core of the present disclosure, a second area 803 having no polymer particles can be formed on the fluffy fabric of the diaper core, thereby improving the urine absorption effect of the fluffy fabric of the diaper core.

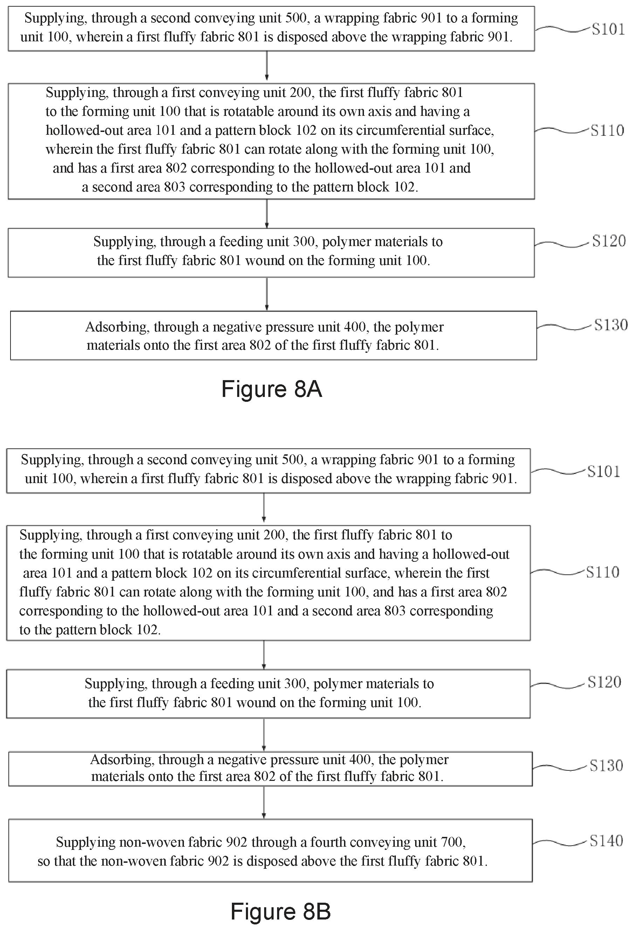

In an exemplary embodiment, as shown in Figure 8A, before supplying, through the first conveying unit 201, the first fluffy fabric 801 to the forming unit 100 (i.e., step S110), the method for manufacturing a diaper core further comprises:
Step S101, supplying, through a second conveying unit 202, a wrapping fabric 901 to the forming unit 100.

Therein, the first fluffy fabric 801 is disposed above the wrapping fabric 901.

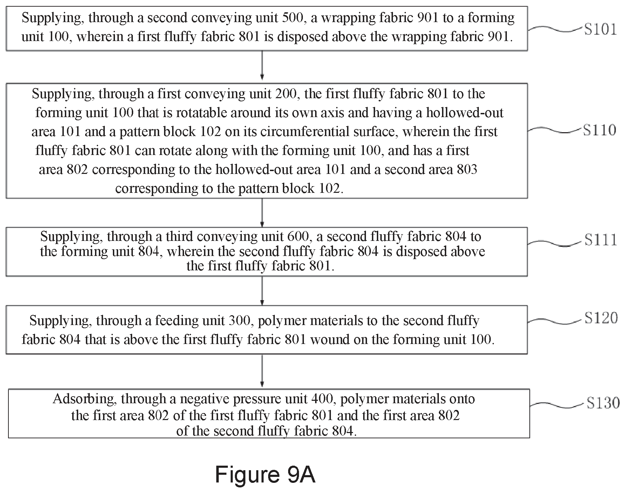

In an exemplary embodiment, as shown in Figure 9A, after supplying, through the first conveying unit 201, the first fluffy fabric 801 to the forming unit 100 (i.e., step S110), the method for manufacturing a diaper core further comprises:
Step S111, supplying, through a third conveying unit 203, a second fluffy fabric 804 to the forming unit 100.

Therein, the second fluffy fabric 804 is disposed above the first fluffy fabric 801. The second fluffy fabric 804 also has a first area 802 and a second area 803.

Step S120 specifically includes: supplying, through the feeding unit 300, polymer particles to the second fluffy fabric 804 that is above the first fluffy fabric 801 wound on the forming unit 100.

Step S130, adsorbing, through the negative pressure unit 400, the polymer particles onto the first area 802 of the first fluffy fabric 801 and the first area 802 of the second fluffy fabric 804.

In an exemplary embodiment, as shown in Figure 8B, after adsorbing, through the negative pressure unit 400, the polymer particles onto the first area 802 of the first fluffy fabric 801 (i.e., step S130), the method for manufacturing a diaper core further comprises:
Step S140, supplying non-woven fabric 901 through a fourth conveying unit 204, so that the non-woven fabric 902 is disposed above the first fluffy fabric 801 (see Figures 4A and 4B for reference).

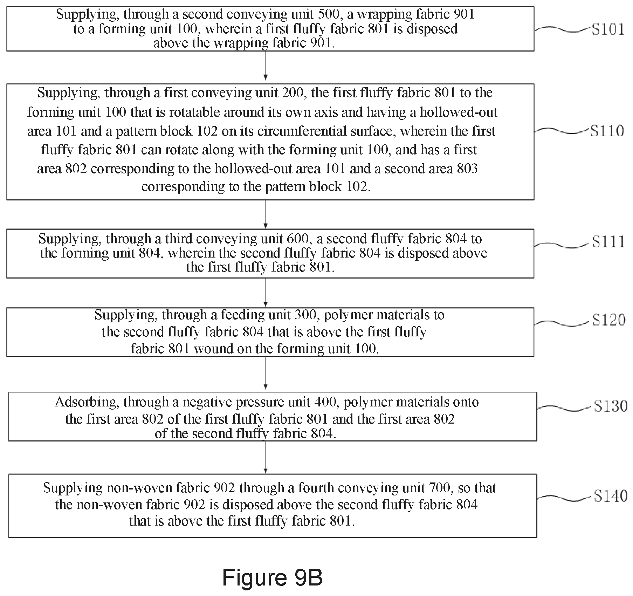

If the second fluffy fabric 804 is supplied, the non-woven fabric 902 is disposed above the second fluffy fabric 804 that is above the first fluffy fabric 801 (see Figures 4A, 4B, and 9B for reference).

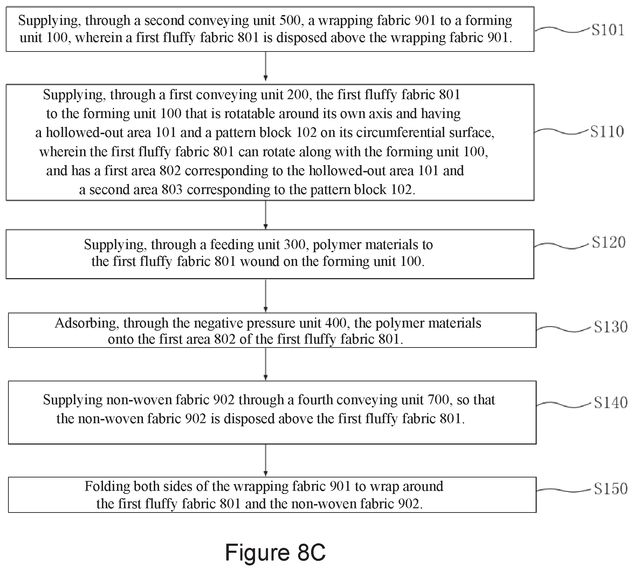

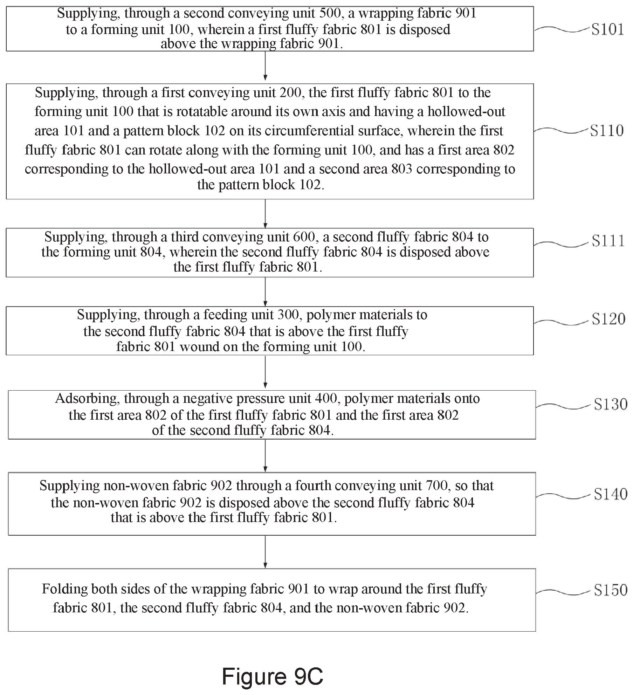

In an exemplary embodiment, after supplying the non-woven fabric 902 through the fourth conveying unit 204, such that the non-woven fabric 902 is disposed above the first fluffy fabric 801, or after supplying the non-woven fabric 902 through the fourth conveying unit 204, such that the non-woven fabric 902 is disposed above the second fluffy fabric 804 that is above the first fluffy fabric 801, the method for manufacturing a diaper core further comprises:
Step S150, folding both sides of the wrapping fabric 901 to wrap around the first fluffy fabric 801 and the non-woven fabric 902 (see Figures 4A, 4B, and 8C for reference), or to wrap around the first fluffy fabric 801, the second fluffy fabric 804, and the non-woven fabric 902 (see Figures 5A, 5B, and 9C for reference).

Specifically, the long side 903 of the wrapping fabric 901 is folded using tools such as mechanical grippers (see Figure 6E for reference).

In an exemplary embodiment, the apparatuses involved in Figures 7 to 9C are the same with those as shown in Figures 1 to 5B, and will not be repeated herein.

Other embodiments of the present disclosure also provide a diaper core, which is manufactured by an apparatus for manufacturing a diaper core as shown in Figures 1 to 3B.

Other embodiments of the present disclosure also provide a diaper core, which is manufactured by a method for manufacturing a diaper core as shown in Figures 7 to 9C.

It should be noted that Figures 4A, 4B, 5A, and 5B only illustrate the approximate shape of each layer and the approximate positional relationship between them. To better distinguish the boundaries between the layers, gaps are illustrated between the layers in Figures 4A, 4B, 5A, and 5B, but in actual products, there are no gaps between the layers.

In an embodiment, as shown in Figure 4A, the second area 803 of the first fluffy fabric 801 is a solid. In another embodiment, as shown in Figure 4B, the second area 803 of the first fluffy fabric 801 is an opening.

In an embodiment, as shown in Figure 5A, the second area 803 of the first fluffy fabric 801 and the second fluffy fabric 804 is a solid. In another embodiment, as shown in Figure 5B, the second area 803 of the first fluffy fabric 801 and the second fluffy fabric 804 is an opening.

When the diaper core is worn on a person after being made into a diaper, the upper side in Figures 4A, 4B, 5A, and 5B is the side close to the skin.

To verify the effectiveness of the diaper core according to an embodiment of the present disclosure, a control experiment is conducted on the diapers. The only difference between the control group and the experimental group is that there is no corresponding second area 803, and all other conditions remain the same. Therein, "L" represents the diffusion length displayed on the surface layer of the diaper during the absorption of 80ml of physiological saline; the longer the diffusion length displayed on the surface layer, the better the absorption effect of the diaper on the physiological saline. "t" represents the time required to absorb 80ml of physiological saline; the shorter the time required, the faster the diaper core absorbs the physiological saline.

| | L (mm) | t (s) | Corresponding layered structure | Shape of the corresponding second area |
|---|---|---|---|---|
| Control Group 1 | 188 | 76 | Figure 4A | no |
| Experimental Group 1 | 205 | 49 | Figure 4A | Figure 6A |
| Experimental Group 2 | 210 | 46 | Figure 4A | Figure 6B |
| Experimental Group 3 | 210 | 50 | Figure 4A | Figure 6C |
| Experimental Group 4 | 216 | 43 | Figure 4A | Figure 6D |
| Control Group 2 | 191 | 66 | Figure 4B | no |
| Experimental Group 5 | 215 | 44 | Figure 4B | Figure 6A |
| Experimental Group 6 | 237 | 51 | Figure 4B | Figure 6B |
| Experimental Group 7 | 211 | 44 | Figure 4B | Figure 6C |
| Experimental Group 8 | 226 | 40 | Figure 4B | Figure 6D |
| Control Group 3 | 199 | 68 | Figure 5A | no |
| Experimental Group 9 | 219 | 49 | Figure 5A | Figure 6A |
| Experimental Group 10 | 216 | 48 | Figure 5A | Figure 6B |
| Experimental Group 11 | 221 | 44 | Figure 5A | Figure 6C |
| Experimental Group 12 | 228 | 42 | Figure 5A | Figure 6D |
| Control Group 4 | 198 | 64 | Figure 5B | no |
| Experimental Group 13 | 223 | 50 | Figure 5B | Figure 6A |
| Experimental Group 14 | 230 | 47 | Figure 5B | Figure 6B |
| Experimental Group 15 | 237 | 40 | Figure 5B | Figure 6C |
| Experimental Group 16 | 229 | 43 | Figure 5B | Figure 6D |

From the table, it can be seen that the diffusion length of the experimental groups with the second area 803 exceeds that of the corresponding control groups, and the absorption rate exceeds that of the corresponding control groups.

For convenience of interpretation and precise limitation of the appended claims, the terms "up", "down", "in", "out", "on", "below", "upside", "downside", "upward", "downward", "front" "back" "backward", "inside", "outside", "inward", "outward" "internal", "external", "internal", "external", "forwards", "backwards" are used to describe the features of the exemplary embodiments with reference to the positions of these features shown in the accompanying drawings.

The foregoing descriptions of specific exemplary embodiments of the present disclosure have been presented for purposes of illustration and exemplification. The preceding description is not intended to be exhaustive, nor is it intended to limit the disclosure to the precise form disclosed. Obviously, many modifications and variations are possible based on the above teachings. The purpose of selecting and describing exemplary embodiments is to explain the specific principles and practical applications of the present disclosure, so that others skilled in the art can implement and utilize various exemplary embodiments of the present disclosure, as well as various alternatives and modifications. The scope of the present disclosure is defined by the appended claims and their equivalents.

## Claims

1. An apparatus for manufacturing a diaper core, comprising:
a forming unit capable of rotating around its own axis and having a hollowed-out area and a pattern block on its circumferential surface;
a first conveying unit capable of supplying a first fluffy fabric to the forming unit and enabling the first fluffy fabric to be wound on the circumferential surface of the forming unit, wherein the first fluffy fabric can rotate along with the forming unit, and has a first area corresponding to the hollowed-out area and a second area corresponding to the pattern block;
a feeding unit capable of supplying polymer particles to the first fluffy fabric wound on the forming unit; and
a negative pressure unit capable of adsorbing the polymer particles onto the first area of the first fluffy fabric.

2. The apparatus for manufacturing a diaper core according to claim 1, further comprising:
a second conveying unit capable of supplying a wrapping fabric to the forming unit.

3. The apparatus for manufacturing a diaper core according to claim 1, further comprising:
a third conveying unit capable of supplying a second fluffy fabric to the forming unit.

4. The apparatus for manufacturing a diaper core according to claim 1, further comprising:
a fourth conveying unit capable of supplying non-woven fabric.

5. The apparatus for manufacturing a diaper core according to claim 1, wherein the feeding unit comprises:
a first feeding hopper capable of supplying first polymer particles to the first fluffy fabric wound on the forming unit; and
a second feeding hopper capable of supplying second polymer particles to the first fluffy fabric wound on the forming unit.

6. The apparatus for manufacturing a diaper core according to claim 1, wherein the pattern block has a curvature.

7. The apparatus for manufacturing a diaper core according to claim 1, wherein the pattern block is integrated with the hollowed-out area.

8. The apparatus for manufacturing a diaper core according to claim 1, wherein the circumferential surface of the forming unit has a through hole, and the pattern block has a plug-in rod corresponding to the through hole and inserted into the through hole so that the pattern block is detachably installed on the circumferential surface of the forming unit.

9. The apparatus for manufacturing a diaper core according to claim 1, wherein the feeding unit is arranged above or diagonally above the circumferential surface of the forming unit.

10. The apparatus for manufacturing a diaper core according to claim 1, wherein the forming unit comprises:
a shaft; and
a housing rotatably mounted to the shaft.

11. A method for manufacturing a diaper core, comprising:
supplying, through a first conveying unit, a first fluffy fabric to a forming unit that is rotatable around its own axis and having a hollowed-out area and a pattern block on its circumferential surface, wherein the first fluffy fabric can rotate along with the forming unit, and has a first area corresponding to the hollowed-out area and a second area corresponding to the pattern block;
supplying, through a feeding unit, polymer particles to the first fluffy fabric wound on the forming unit; and
adsorbing, through a negative pressure unit, the polymer particles onto the first area of the first fluffy fabric.

12. The method for manufacturing a diaper core according to claim 11, wherein before supplying, through the first conveying unit, the first fluffy fabric to the forming unit, the method for manufacturing a diaper core further comprises:
supplying, through a second conveying unit, a wrapping fabric to the forming unit;
wherein the first fluffy fabric is disposed above the wrapping fabric.

13. The method for manufacturing a diaper core according to claim 11, wherein after supplying, through the first conveying unit, the first fluffy fabric to the forming unit, the method for manufacturing a diaper core further comprises:
supplying, through a third conveying unit, a second fluffy fabric to the forming unit;
wherein the second fluffy fabric is disposed above the first fluffy fabric.

14. The method for manufacturing a diaper core according to claim 11, wherein after adsorbing, through the negative pressure unit, the polymer particles onto the first area of the first fluffy fabric, the method for manufacturing a diaper core further comprises:
supplying non-woven fabric through a fourth conveying unit, so that the non-woven fabric is disposed above the first fluffy fabric.

15. The method for manufacturing a diaper core according to claim 11, wherein the feeding unit comprises:
a first feeding hopper capable of supplying first polymer particles to the first fluffy fabric wound on the forming unit; and
a second feeding hopper capable of supplying second polymer particles to the first fluffy fabric wound on the forming unit.

16. The method for manufacturing a diaper core according to claim 11, wherein the feeding unit is arranged above or diagonally above the circumferential surface of the forming unit.

17. A diaper core manufactured by the apparatus for manufacturing a diaper core according to any one of claims 1-10.

18. A diaper core manufactured by the method for manufacturing a diaper core according to any one of claims 11-16.
